Europäisches Patentamt

(19) European Patent Office

Office européen des brevets

(11) EP 0 436 562 B1

(12) EUROPEAN PATENT SPECIFICATION

(45) Date of publication and mention
of the grant of the patent:
15.01.1997 Bulletin 1997/03

(51) Int Cl.[6]: C12P 1/02, C12P 23/00,
C12N 1/16, C12R 1/645

(21) Application number: 89909294.4

(22) Date of filing: 07.08.1989

(86) International application number:
PCT/US89/03327

(87) International publication number:
WO 90/01552 (22.02.1990 Gazette 1990/05)

(54) **Process for in vivo production of astaxanthin and Phaffia rhodozyma yeast of enhanced astaxanthin content**

Verfahren zur In vivo-Produktion von Astaxanthin und Phaffia rhodozyma Hefe mit erhöhtem Astaxanthingehalt

Procédé de production in vivo d'astaxanthine et levure Phaffia rhodozyma à teneur en astaxanthine augmentée

(84) Designated Contracting States:
AT BE DE FR GB IT NL SE

(30) Priority: 08.08.1988 US 229536

(43) Date of publication of application:
17.07.1991 Bulletin 1991/29

(73) Proprietor: IGENE BIOTECHNOLOGY, INC.
Columbia, MD 21045 (US)

(72) Inventors:
• JOHNSON, Eric, A.
Madison, WI 54711 (US)
• SCHREIBER, David
Columbia, Maryland 21045 (US)
• HO, Kwok, P.
San Diego, California 92129 (US)
• HALL, William, T.
Rockville, MD 20853 (US)
• YANG, Huei-hsiung
Rockville, MD 20853 (US)
• GELDIAY-TUNCER, Beril
College Park, MD 20740 (US)

(74) Representative:
Rickard, Timothy Mark Adrian et al
Brookes & Martin,
High Holborn House,
52/54 High Holborn
London WC1V 6SE (GB)

(56) References cited:
• See also references of WO9001552
• American Type Culture Collection, Catalogue of Fungi/Yeasts, 17th edn., 1987, page 265
• Dictionary of Antibiotics and Related Substances, London-New-York: B. W. Bycroft, 1988, 127-8

**Description**

FIELD OF THE INVENTION

The invention pertains in one aspect to an economical process for in vivo production of the pigment astaxanthin. In another aspect, the invention pertains to a process for enhancing astaxanthin content of cultures of microorganisms of genus Phaffia, the process comprising culturing a microorganism of genus Phaffia in a nutrient medium containing an antibiotic, a cytochrome B inhibitor, or a terpenoid synthetic pathway inhibitor, cultivating surviving pigment enhanced microorganisms, and harvesting the yeast.

BACKGROUND OF THE INVENTION

The reddish carotenoid pigment astaxanthin is commonly found in nature and is conspicuously displayed by a number of animals. Animals unable to synthesize this pigment rely on dietary intake of this pigment or a pigment precursor.

The red skin and flesh color of naturally occurring salmon and trout is due primarily to astaxanthin, which is usually present as an unbound pigment in these fishes. In nature, marine zooplankton and nekton in the diet provide salmon with their carotenoid pigments.

Due to a lack of dietary astaxanthin, fish raised on fish-farms or in hatcheries are generally pale and lack the skin and flesh colors characteristic of fish grown in their natural environment. Whether or not the carotenoids are nutritionally important in the animal or human diet has not been determined, but pigments do make certain foods attractive. That is, since the color of a food is frequently an indicator of its quality, there is a strong consumer preference for fish having natural coloration, even though nutritionally the pale farm produced fish may be identical to those grown in their natural environment. There is also evidence that astaxanthin or its precursor contributes to the distinctive flavor of baked salmon.

Recent increasing concern over health risks has resulted in a ban on various synthetic coloring agents which have a potential of carcinogenicity and/or teratogenicity. Yellow and red azo dyes which are increasingly prohibited from use in foods are being replaced with non-toxic carotenoids. The carotenoids are generally not toxic even at high levels. Thus, naturally occurring carotenoids are the preferred pigment for coloring, e.g., salmonids.

In the past, numerous studies have been carried out utilizing carotenoid containing crustaceans or crustacean processing wastes in salmonid diets. The pale color of fish produced on fish-farms or in hatcheries is improved when the fish are fed a diet supplemented by large quantities of dried, ground-up exoskeletal crustacean remains. Crustacean shells are, however, very low in carotenoid content and high in minerals which, without extensive processing to improve their dietary quality, restricts their inclusion in salmonid diets. Further, a satisfactory color can be developed in this manner of feeding only over long periods of time and it is desirable, if not indeed essential in the economic sense, to develop satisfactory colors within very short periods of time.

It is known that astaxanthin per se can be added to fish food to improve fish color. For example, United States Patent 4,239,782 describes a method for enhancing the color of fish comprising adding to the fish food a pigmenting agent such as astaxanthin and additionally limited amounts of the hormone testosterone, which hormone acts as a catalyst in combination with a chosen pigmenting agent or agents to enhance the color of fish.

The two primary commercial sources for astaxanthin per se are extracts from crustacean shells and chemical synthetics. The red carotenoid pigment can be extracted from the exoskeletal crustacean shells and tissues and fed, admixed with other feed in dietary formulations, to the farm fish, crustacea and certain fowls in massive concentrations to develop satisfactory skin, flesh, carapace or egg yolk pigmentations. Examples of processes for extraction of astaxanthin from crustacean shell and tissue waste are described, for example, in United States Patents 3,906,112 (Anderson) and 4,505,936 (Meyers et al).

In an article in Journal of Food Science, Volume 47 (1982), entitled "Extraction of Astaxanthin Pigment from Crawfish Waste Using a Soy Oil Process", various extraction techniques are described. For example, whole crawfish waste is ground-up, the comminuted crawfish waste is admixed with water, the pH is adjusted with an alkali or acid, an enzyme is added to the solution, and the solution stirred, heated and hydrolyzed. After hydrolysis, the astaxanthin is extracted with oil and the astaxanthin enriched oil recovered by centrifugation. However, the cost of natural isolates of astaxanthin, especially from krill and crawfish shells, can cost anywhere from $5,000 to $15,000 per kilogram. Obviously, a less source dependent and more economical process for production of astaxanthin is needed.

Pigmentation of salmon and trout flesh has also been accomplished using the synthetic carotenoid canthaxanthin as a feed additive, but this chemical is rather expensive and has been reported to produce a somewhat unsatisfactory color in salmonids. Recent work in chemical synthesis of astaxanthin is exemplified by United States Patents 4,245,109 (Mayer et al), 4,283,559 (Broger et al), and 4,585,885 (Bernhard et al). The present cost of synthetic astaxanthin pigment is approximately $2,000 per kilogram. Many countries, however, prohibit the use of synthetic carotenoids.

Astaxanthin remains one of the most expensive ingredients used in salmon feed for pen-reared salmon. As the interest in aquaculture, i.e., farming fish, has exploded recently, the commercial demand for an economical source of astaxanthin has grown proportionately.

The pigmentation of avian egg yolks has also been studied because of the economic importance of color in chicken egg yolks. Yolks with a high pigment content are demanded. The most common pigment source in commercial diets has been yellow corn, which supplies the prominent egg yolk pigments cryptoxanthin, zeaxanthin and lutein. Unfortunately, higher energy grains such as milo, wheat, rice and barley are replacing corn in the chicken diet, with the consequent loss in pigmentation. Astaxanthin can be used as a poultry food supplement to increase yolk pigmentation.

One approach not presently commercially employed in production of astaxanthin is biosynthesis, i.e., employment of microorganisms to synthesize astaxanthin.

As a microbial source of astaxanthin, the yeast Phaffia rhodozyma is known (Johnson, "Astaxanthin Production by the Yeast Phaffia rhodozyma and its use as a Pigment Source in Animal Feeding", Masters Thesis, University of California at Davis, 1976). Yeast is generally considered to be a highly nutritious feedstuff and is often desirable in animal diets; the additional attribute of containing astaxanthin suggests that P. rhodozyma may be an ideal feed supplement for animals that require a dietary source of this pigment, e.g. salmonids, crustaceans, laying hens, or birds such as flamingoes.

However, the pigment yield of naturally occurring P. rhodozyma is only in the order of 200 to 600 ppm/dry weight of yeast in 6 day growth. As a source of astaxanthin per se, naturally occurring P. rhodozyma is inadequate. Experiments in supplementing the diets of certain fish, crustaceans and fowl with naturally occurring P. rhodozyma showed some promise. Practically, however, the large volume of P. rhodozyma which must be added as a food supplement in order to obtain satisfactory levels of pigmentation detracts from the commercial suitability of naturally occurring P. rhodozyma as a pigment source.

## SUMMARY OF THE INVENTION

Accordingly, it is an object of the present invention to provide economical process for in vivo production of the pigment astaxanthin.

Another object of the present invention is to provide a process for obtaining cultures of the yeast Phaffia rhodozyma having increased astaxanthin content. Another object of the present invention is to provide P. rhodozyma characterized by a high astaxanthin content.

It is another object of the present invention to develop a process for improving astaxanthin content of progeny derived from naturally occurring P. rhodozyma such as strain ATCC-24230 or ATCC-24202 deposited with the ATCC.

Yet another object of the present invention is to develop a process for improving astaxanthin content of mutated strains of the yeast P. rhodozyma.

These and other objects have been attained by a process comprising, in it's most basic form a process for the production of a yeast having an enhanced astaxanthin content comprising culturing, in a nutrient medium containing an antibiotic selected from antimycin A, tunicamycin and nystatin, cytochrome B inhibitor, or a terpenoid synthetic pathway inhibitor, a microorganism of genus Phaffia.

The key step in strain development is the morphological selection step. There is no limit to the possible combinations of sequence and type of mutation and antibiotic, a cytochrome B inhibitor, or terpenoid inhibitor selection to which P. rhodozyma may be subject within the present invention. Recent results confirm the reproducibility of this technique.

Related objects and advantages of the present invention will become more apparent by reference to the following figures and detailed description.

## BRIEF DESCRIPTION OF THE FIGURES

The process in accordance with the present invention will be described with reference to the accompanying drawings, in which:

Fig. 1 shows the chemical structure and relationship of various pigments and intermediates found in P. rhodozyma.

Fig. 2 is a flow chart showing preferred sequences of mutant isolations in P. rhodozyma.

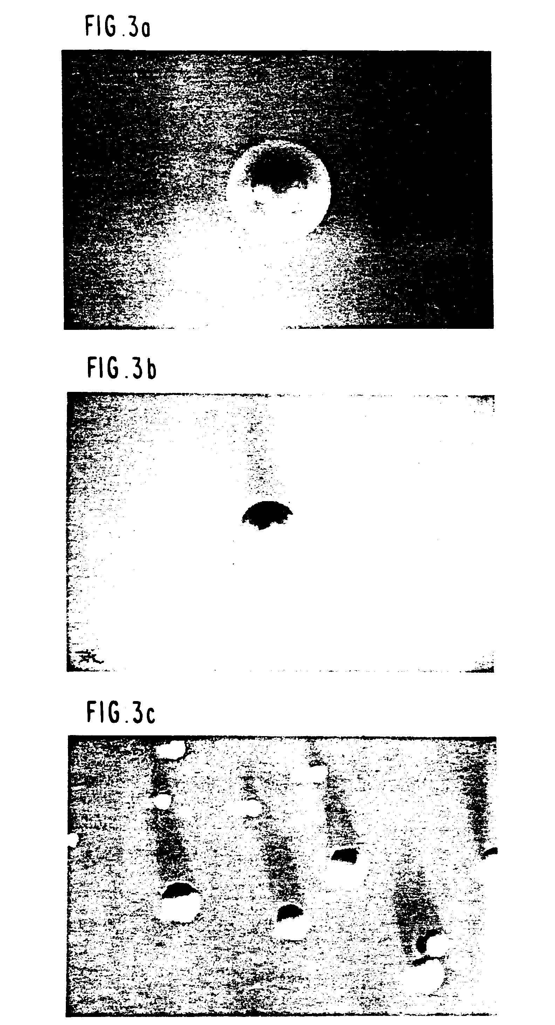

Fig. 3 is a micropictograph showing the appearance of variegated colonies.

## DETAILED DESCRIPTION OF THE INVENTION

During an expedition in 1967 for the purpose of studying yeast florae associated with trees on the major Japanese Islands and in the Pacific Northwest, a peculiar astaxanthin synthesizing organism was isolated from slime fluxes of various broad-leafed trees (Phaff et al., "A comparative study of the yeast florae associated with trees on the Japanese Islands and on the west coast of North America", Proc. IV: Ferment. Technol. Today, (1972) pp. 759-774). This organism, now known as Phaffia rhodozyma, was determined to have the capabilities of producing carotenoid pigments and fermenting several sugars.

Phaffia has been granted a genus because of its unique characteristics, which include its ability to ferment glucose and other sugars (compared to the other carotenoid-forming yeasts which are all strictly aerobic), its synthesis of astaxanthin as its principal carotenoid, its mode of bud formation, its possession of certain metabolic properties such as the ability to use urea which is less common in ascomycetous yeasts, and its cell wall composition. P. rhodozyma, the only species in this genus, reproduces exclusively by budding, and an examination of this mode of vegetative reproduction by scanning electron microscopy reveals that the yeast buds repeatedly from the same site which leads to the formation of the multilayered cell wall.

Other characteristics of Phaffia include its formation of chlamydospores, its mol % G+C content of 48.3%, its ability to split urea by urease, and its apparent lack of a perfect stage. Its properties suggest that the yeast is of basidiomycetous affinity. Attempts to find the sexual cycle of Phaffia have all failed.

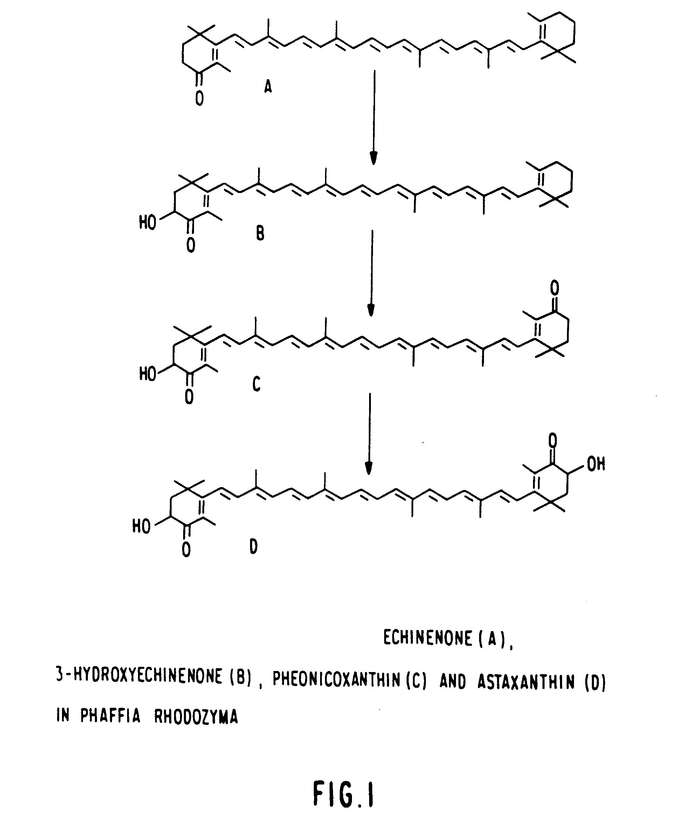

Andrewes et al, as reported in "Carotenoids of Phaffia rhodozyma, a red pigmented fermenting yeast", Phytochem. 15, (1976) pp. 1003-1007, found the carotenoids of P. rhodozyma to be unusual. Pigments and pigment intermediates found by Andrewes in naturally occurring P. rhodozyma are shown in Fig. 1 where (A) is echinenone, (B) is 3-hydroxyechinenone, (C) is phenicoxanthin, and (D) is astaxanthin. Astaxanthin was found to be the major pigment synthesized by this yeast; in the naturally occurring yeast it comprised approximately 85% of the carotenoid mixture.

While the carotenoids of most plant and animal sources can be readily extracted with water-miscible solvents, the yeasts are well known for the tenacious attachment of their pigments. Astaxanthin is firmly attached in the yeast cell and not extractable by lipid solvents unless the structure of the yeast cell is first altered.

The most quantitative method of extraction involves mechanically breaking the yeast cells such as in a French press (Simpson et al., "Modified French press for the disruption of microorganisms", J. Bact., 86, (1963) pp. 1126-1127) or in a Braun homogenizer (Bae et al., "The occurrence of plectaniaxanthin in Cryptococcus laurentii", Phytochem., 10, (1970) pp. 625-629), and then extracting the cells with solvents. These methods are used routinely for estimation of astaxanthin concentration.

The need for the large scale recovery of astaxanthin led to the development of an enzymatic method of extraction. The method utilized extracellular lytic enzymes produced by the bacterium Bacillus circulans WL-12, which partially digested the yeast cell wall and rendered the carotenoid pigments extractable by lipid solvents. Complete extraction of astaxanthin from heat-killed P. rhodozyma cells was obtained after growing B. circulans WL-12 on these yeast cells for 26 hours and then extracting the yeast-bacterium mixture with acetone.

A bacteria-free lytic system, which gave quantitative extraction of astaxanthin from P. rhodozyma, was obtained by concentrating the culture broth from the growth of B. circulans WL-12 on P. rhodozyma cells. Preferably, B. circulans WL-12 is grown on a medium containing P. rhodozyma cells. The lytic system was found to work most efficiently at pH 6.5 and with low concentrations of yeast.

Generally, nutrition and environment have an effect on carotenoid yield in various carotenoid producing microorganisms. Enhancement of carotenoid formation in fermentations in complex media has been reviewed (Hanson, "Microbial production of pigments and vitamins", in Microbial Technology, edited by H.J. Peppler, Reinhold, New York, (1967) pp. 222-250).

Carotenoids are tetraterpenes and their basic pathway of synthesis is similar to that of other terpenoids. Acetyl CoA is the initial precursor and the first specific terpenoid compound is mevalonic acid (Schopfer et al, "Sur la biosynthése du β-caroténe par Phycomyes cultivé sur un mileu contenant de l'acétate de sodium comme unique source de carbone", Experientia, 8, (1952) p. 140). Isopentenyl pyrophosphate is the fundamental precursor from which carotenoids are derived (see Goodwin, "Biosynthesis", Carotenoids, Edited by O. Isler, Basel, Birkhauser, (1971) pp. 577-636; Britton, "Biosynthesis of Carotenoids", in Chemistry and biochemistry of plant pigments, Vol. 1, (1976) pp. 262-327).

Most previous work with P. rhodozyma has been done with the type strain #67-210. Visual examination of young colonies of several additional natural isolates (UCD-FST #s 67-202, 67-203, 67-210, 67-383, 67-385 and 68-653C) indicated that 67-210 and 67-385 (American Type Culture Collection ATCC Nos. 24202 and 24230 respectively) were the most highly pigmented strains. Quantitative determination of astaxanthin content in these two strains after 5 days growth in YM broth indicated that 67-385 contained ≈450 μg/g dry yeast in comparison with ≈295 μg/g in 67-210. 67-385 was primarily employed by the inventors for strain development because of its higher natural astaxanthin content.

As a result of a series of experiments designed to determine the optimal culture conditions for growth and pigmentation of P. rhodozyma, it was found that astaxanthin biosynthesis occurred maximally during the exponential phase of growth. The pigment yield in the growth medium was found not to be solely dependent on cell concentration but was influenced by the culture conditions. The optimal pH for astaxanthin production was found to be 5.0 in shake flasks. At the other pHs tested, however, the concentration of astaxanthin in P. rhodozyma remained relatively constant.

The temperature of cultivation was found to influence the growth rate of P. rhodozyma but not the accumulation of astaxanthin in the yeast cell. The effects of light also did not affect carotenogenesis in P. rhodozyma, although the

EP 0 436 562 B1

cells grown under high light intensity appeared to have a redder hue, which may have been due to different concentrations of particular carotenoids.

It was found that a low glucose concentration and high air supply promoted efficient astaxanthin production by P. rhodozyma. The concentration of astaxanthin in the red yeast decreased considerably if the air supply was below 20 mmoles/h or the glucose concentration was above 4% w/v. However, astaxanthin was still the predominant pigment in yeast cultured under either of these conditions. If the effects of low air and high glucose were combined, however, then the astaxanthin concentration in P. rhodozyma was decreased to extremely low levels and the formation of β-zeacarotene occurred. Under these adverse conditions, again, astaxanthin was not efficiently formed from the carotenes.

When cultured on carbon compounds which repressed ethanol production in this yeast (e.g. cellobiose) the yields of astaxanthin were comparatively high. If cultured on carbon sources which presumably promoted ethanol production (e.g. high glucose) the astaxanthin yields were comparatively low. Carbon compounds metabolized via the pentose-phosphate pathway (e.g. xylose) did not promote efficient carotenogenesis.

Despite extensive experimenting in optimizations of nutrient medium and environmental conditions on naturally occurring strains of P. rhodozyma, a dramatic increase in pigment content was not attained. The inventors concluded that manipulation of the above discussed factors alone may not be sufficient to induce increase in astaxanthin content of P. rhodozyma to the extent necessary to render biosynthesis commercially feasible.

With the objective of isolating a novel genetic mutant of P. rhodozyma capable of increased astaxanthin production, P. rhodozyma was subject to mutagenesis. However, results were inconsistent, presumably due to the random and non-directed nature of mutation. For example, attempts at isolating highly pigmented variants were not successful after screening several thousand colonies after UV exposure. Most of the UV-generated mutants were considerably reduced in astaxanthin content and were very pale.

Potential for further progress in improvement in astaxanthin content in P. rhodozyma by conventional mutagenic techniques appears to be limited. This is because microorganisms possess tight genetic regulatory controls over the biosynthesis of their cellular components so that they tend not to overproduce unnecessary cellular constituents. Since the biosynthesis of pigment such as astaxanthin is apparently tightly regulated in any given strain of yeast cells, the chances of producing a viable inheritable genetic alteration to produce colonies capable of increased astaxanthin production by undirected mutagenesis is probably very low.

Since screening of colonies after mutagenesis was relatively unsuccessful, the inventors tried to develop selection procedures for astaxanthin overproduction. Because astaxanthin formation is decreased by high concentrations of glucose, the inventors tried 2-deoxyglucose as a selective agent to isolate glucose-resistant strains that might be catabolite-derepressed and, in turn, exhibit enhanced pigment biosynthesis. Although some strains generated by this selective procedure were changed in pigmentation, many were extremely unstable and none were increased in astaxanthin concentration more than two-fold over the naturally occurring parent.

Inhibitors of sterol biosynthesis including ketoconazole and miconazole were incorporated into yeast malt extract medium agar, which resulted in significant killing of P. rhodozyma. Screening of several thousand survivors, however, did not produce highly pigmented yeast strains. Several other compounds tested including nicotine (1 mM), imidazole (4 mM), 2-methylimidazole (1 mM), and morpholine (10 mM) did visually change the color of the colonies, but did not impair growth, which indicated a change in carotenoid composition. However, since these variants were not increased in astaxanthin, no further analysis in the changes in pigment compositions was carried out.

The inventors also tried treating P. rhodozyma with several inhibitors of electron transport including thenoyltrifluoroacetone (TTFA), antimycin A, and sodium cyanide. Naturally occurring P. rhodozyma was substantially killed by low concentrations of antimycin A and TTFA, but was found to be relatively insensitive to cyanide and azide.

As a result of extensive experimentation, discussed above in part, the inventors made a surprising discovery of a process by which colonies of P. rhodozyma can be produced, which colonies are characterized by a high astaxanthin content. P. rhodozyma produced by the process of the present invention is non-reverting, the astaxanthin is present in the yeast in a sufficiently high concentration to be a more effective dietary food and pigment supplement than naturally occurring P. rhodozyma, and colonies of the yeast can be obtained which are able to produce astaxanthin in sufficient quantities to render astaxanthin fermentation a commercially feasible process for production of astaxanthin.

More specifically, the inventors have discovered that by subjecting a naturally occurring or mutated strain of P. rhodozyma to morphological selection using a selecting agent, and particularly an antibiotic, a cytochrome B inhibitor, or a terpenoid synthetic pathway inhibitor as a selecting agent, a directed and specific selection of highly pigmented P. rhodozyma takes place.

The selected strain may be subjected furthermore to growth in the presence of a main respiratory pathway inhibitor and in the presence of an agent or under the influence of an environmental condition which triggers a secondary oxidative pathway.

For example, plating of the pink yeast, P. rhodozyma, to agar containing an antibiotic, a cytochrome B inhibitor, or a terpenoid synthetic pathway inhibitor gave rise to colonies of unusual morphology characterized by a non-pigmented

5

lower smooth surface that developed highly pigmented vertical papillae after 1 to 2 months. Isolation and purification of the papillae, followed by testing for pigmentation in shake flasks, demonstrated that several mutants were increased 3 to 6 fold in astaxanthin content compared to the parental natural isolate.

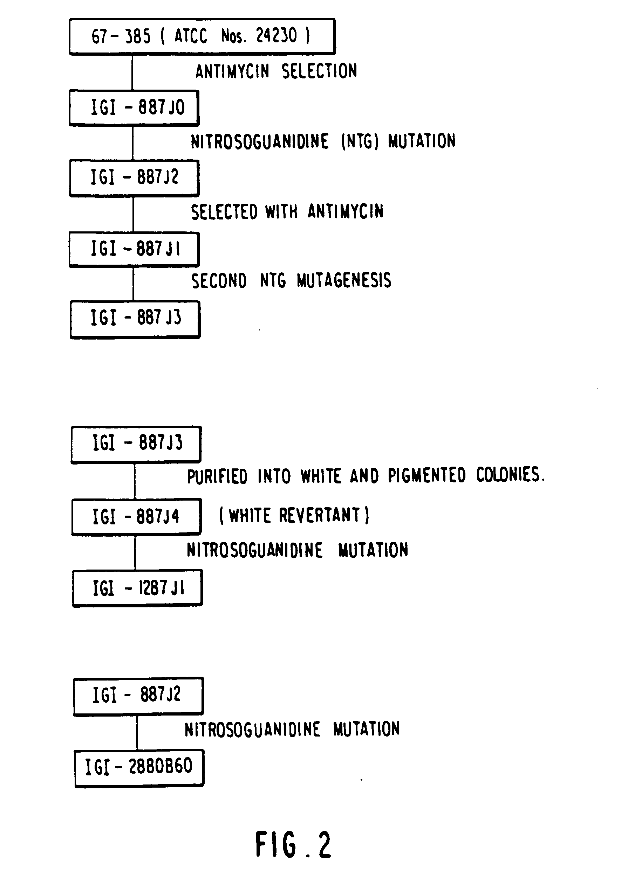

One of the selected progeny ((IGI-887J0; see Fig. 2 and accompanying text) was characterized physiologically. The mutant grew slower on various nitrogen sources and had lower cell yields on several carbon sources. It showed increased sensitivities to the respiratory inhibitors such as antimycin A and TTFA, but did not differ from the natural isolate in sensitivity to cyanide or azide. It was more sensitive to killing by hydrogen peroxide. Analysis of the carotenoids by thin layer chromatography showed two unknown carotenoids not present in the parent, as well as an increased accumulation of carotenes and cisastaxanthin.

Antibiotic selection agents are selected from one or more of antimycin A. tunicamycin and nystatin. Antimycin may also be classified as an electron transport inhibitor or a cytochrome B inhibitor. Other cytochrome B inhibitors which enhance pigmentation of select progeny include, for example, 2-n-heptyl-4-hydroxy-quinoline-N-oxide HOQNO). The terpenoid synthetic pathway inhibitors include, for example mevalonic acid lactone, which may be generally referred to as a metabolic inhibitor, and which is an example of a sterol inhibitor.

Concentrations of selection agents, e.g., antimycin A, preferably range from 1 to 100 μM on yeast malt extract medium (YM) plates, more preferably 30 to 80 μM, and most preferably, for generation of the most distinctive colonies, are approximately 50 μM.

The effect of the antibiotic, cytochrome B inhibitor, or terpenoid synthetic pathway inhibitor on P. rhodozyma is surprising and the specific underlying mechanism has yet to be elucidated. It is surprising that P. rhodozyma subjected to antibiotic, cytochrome B inhibitor, or terpenoid synthetic pathway inhibitor selection produce colonies of higher pigment contents, while experiments conducted with other agents, such as respiratory inhibitors including KCN, rotenone, TTFA and others, do not seem to significantly influence pigmentation of P. rhodozyma on YM plates or in liquid growth media.

The inventors have developed a hypothesis which may provide a possible explanation for the effect of antimycin A on P. rhodozyma, but this hypothesis is speculative and should not be taken to affect the scope of the invention.

The inventors suspect that since ring closing and hydroxylation may depend on cytochrome P450 as occurs in sterol synthesis, and a cytochrome B inhibitor such as antimycin A reacts with cytochrome B which is the same cytochrome that reduces cyt. P450, perhaps the increased pigmentation in the antimycin A mutants is due to altered cyt. P450 function or activity. Also this molecule is known to inactivate glutamine synthetase and this may be the reason for an observed slowing of nitrogen catabolism.

Recent results confirm that this technique can work repeatedly with P. rhodozyma.

The naturally occuring or antibiotic, cytochrome B inhibitor, or terpenoid synthetic pathway inhibitor selected yeast cells may additionally be exposed to a mutagen before, after, or before and after selection, or in any desired number or combination of mutation and selection steps, so long as antibiotic, cytochrome 3 inhibitor, or terpenoid synthetic pathway inhibitor selection is included in the protocol.

Mutagens or mutagenic agents can be selected from a variety of chemicals or other exposures, and are preferably powerful enough to cause production of nonrevertable mutations. Mutants wherein no degeneration has been observed are highly desirable for industrial uses. Strong mutagens include ethylmethane sulfonate, nitrosoguanidine (N-methyl-N-nitro-N-nitroso-guanidine), nitrous acid (though relatively high amounts may be needed), ultraviolet radiation in significant amounts, x-ray, and others.

Presently preferred are nitrosoguanidine and UV because of their ready availability, their relatively powerful mutagenic nature, and their relative ease and safety of handling. However, any strong mutagen can be employed, using amounts and exposure techniques as known in the art.

In some cases a weak mutagen can be combined with a strong mutagen. Among the weak mutagens are 2-aminopurine, t-bromouracil, hydroxylamine, sodium bisulfite, and others.

The sequence of mutations and selections with which the highest astaxanthin yielding strains to date have been produced is shown in Fig. 2. With reference to Fig. 2, the naturally occuring parent 67-385, obtained from the American Type Culture Collection ATCC No. 24230, was assayed as containing 200-600 μg/g dry weight of yeast in 6 day growth. 67-385 was subjected to antimycin A selection, and higher pigmented progeny, referred to as IGI-887JO were obtained. The first mutant papillus dissected from the basal colony contained approx. 700 μg total carotenoid/g yeast compared to the parental strain which had 300 to 400 μg/g. IGI-887JO was assayed to have an astaxanthin content of 960 μg/g dry weight of yeast in 6 day growth. IGI-887JO was replated to YM agar containing 50 μM antimycin and second generation antimycin colonies were selected. One of these produced 1200 μg/g and another produced 1450 μg/g. Therefore, it appeared that antimycin A is an excellent selective agent for isolating pigmented variants of P. rhodozyma.

IGI-887JO were subject to nitrosoguanidine (NTG) mutation and IGI-887J2 were obtained which produced 1200 to 1500 μg total carotenoids/g yeast. IGI-887J2 were further selected with antimycin to produce IGI-887J1 which was assayed to have an astaxanthin content of 700-1100 μg/g dry weight of yeast in 6 day growth. A second NTG mutagenesis produced IGI-887J3 progeny.

Upon purification of colonies of IGI-887J3 the isolates segregated into white and pigmented colonies. The white revertant was designated IGI-887J4. The pale colonies grew very poorly on ammonium sulfate at 5 g/l, grew a little better on high levels of ammonium sulfate (20g/l), and on plates grew well on glutamate or glutamine. These results suggest that the strains are progressively impaired in nitrogen metabolism which limits growth rate.

IGI-1287J1 was isolated after nitrosoguanidine mutation of IGI-887J4, the white segregant of IGI-1887J3. ICI-1287J1 was assayed as containing 900-1400 µg/g dry weight of yeast in 6 day growth. This strain also was impaired in nitrogen metabolism. It was noted that strains IGI-887J3 and IGI-1287J1 do not grown on ethanol, while the prior mutants do.

IGI-2880B60 was obtained from IGI-887J2 by NTG mutagenesis, and was assayed to contain 1700 µg/g dry weight of yeast in 6 day growth.

Characterization of the antimycin mutants.

The parental strain (67-385), IGI-887JO, and IGI-887J2 had approximately equal yields of cells with ammonium, glutamate or glutamine as nitrogen sources. However, analysis of growth rates showed that the series of strains grew progressively slower on these nitrogen compounds. These data suggested that the rate of efficiency of nitrogen utilization was impaired in the antimycin A mutants.

The parent and two studied mutants had reduced cell yields on four carbon sources tested, but were more highly pigmented. The antimycin A mutants still fermented glucose and also had significant ethanol dehydrogenase activity. However, IGI-887J2 no longer grew on ethanol and had reduced cell yields on other respiratory substrates including succinate. The reduced yields on the energy sources and the specificity of inhibition by antimycin A for the electron transport chain suggested that respiration or mitochondrial function in the pigmented mutants was altered.

Sensitivities of the mutants to various respiratory inhibitors including antimycin A, theonyltrifluoroacetine, sodium azide, hydrogen peroxide and sodium cyanide were examined. These inhibitors affect different regions of the respiratory chain. Surprisingly, the antimycin A-induced mutants were more sensitive to antimycin A on YM plates and also in liquid media. The parental strain had approximately 50% survival at 100 µM antimycin A, compared to the mutants which did not survive about 60 µM. The concentratins of antimycin A that killed 50% of the populations of IGI-887JO and IGI-887J2 on YM agar were approximately 18 and 3 µM, respectively. The mutants were more sensitive in liquid media and were killed at antimycin A concentrations near 0.5 µM.

These results clearly show that the pigmented papillae that arose from the pale colonies were actually more sensitive to the drug even though they were isolated on plates containing antimycin A. Apparently, the spatial separation of the papillae from the agar facilitated the generation of the more sensitive strains.

The mutants were also quite sensitive to other respiratory inhibitors including TTFA and hydrogen peroxide, but were only slightly more sensitive to cyanide, and did not differ in sensitivity to azide. These data supported the above hypothesis that the antimycin A-isolated strains possessed an altered respiratory chain, and suggested that a lesion may occur in the early regions of the chain, near cytochrome b.

It is necessary to supply suitable amounts of nutrients and minerals in the feed media in order to assure proper microorganism growth, to maximize assimilation of the carbon energy source the cells in the microbial conversion process, and to achieve maximum cellular yields with maximum cell density in the fermentation media.

The composition of the ferment can vary over a wide range, depending in part on the yeast strain, the substrate employed, and the minerals content in the ferment (that is, liquid plus cells). Set forth in the table below are the minimum, broad, and presently preferred ranges of concentrations of various elements in the ferment, the concentration being expressed as of the element, though it is recognized that all or part of each can be present in the form of a soluble ion, or in cases such as P, in a combined form of some type such as phosphate. The amount of each element is expressed in grams or milligrams per liter of ferment (aqueous phase, including cells):

| Weight of Element per Liter of Ferment | | | |
|---|---|---|---|
| Element | Minimum | Broad Range | Preferred Range |
| P | 0.2 g | 0.2-5 g | 0.4-2 g |
| K | 0.1 g | 0.1-3 g | 0.1.0.7 g |
| Mg | 0.15 g | 0.15-3 g | 0.3-1.2 g |
| Ca | 0.06 g | 0.06-1.6 g | 0.08-0.8 g |
| S | 0.1 g | 0.1-8 g | 0.2-5 g |
| Fe | 0.5 mg | 0.5-30 mg | 0.6-20 mg |
| Zn | 2 mg | 2-100 mg | 3-40 mg |
| Cu | 0.6 mg | 0.6-16 mg | 1-10 mg |

(continued)

| Weight of Element per Liter of Ferment | | | |
|---|---|---|---|
| Element | Minimum | Broad Range | Preferred Range |
| Mn | 0.6 mg | 0.6-20 mg | 0.9-8 mg |

The manner in which to carry out the present invention, and further features and advantages of the present invention, will be apparent from the following illustrative Examples. The Examples are provided for the purpose of promoting an understanding of the principles of the invention. Although the conditions and language in the examples is specific, it will nevertheless be understood that no limitation of the scope of the invention is thereby intended, such alterations and further modifications and applications of the basic principles of the present invention as illustrated therein being contemplated as would normally occur to one of ordinary skilled in the art to which the invention relates.

In the Examples, naturally occurring strains are obtained from the official depository, the American Type Culture Collection in Rockville, Maryland, and given ATCC Nos. 24230 and 24202. The ATCC designations reflect that two agar slant cultures of each strain have been deposited with the official depository.

Mutagenesis

Example 1: Ultraviolet Mutagenesis

Strain IGI 2880B60 was mutagenized with ultraviolet light from a germicidal UV lamp at a distance of 12 inches from a saline suspension of cells for 1 min. (95.3% kill), 3 min. (99.4% kill), and 5 min (99.99% kill). Mutagenized cells were kept in the dark for 1 hr. following the period of mutagenesis and plated on yeast malt extract medium (YM) at several dilutions. Plates were incubated at 20°C. Dark red orange colonies were isolated on the basis of observed pigmentation differences and streaked to slants.

Example 2: Nitrosoguanidine Mutagenesis

Strain 1287J1 was grown in YM for 48 hr. Cells were pelleted by centrifugation, the supernatant discarded, and the cells resuspended in 0.1 M citrate buffer, pH 5.0. Cells were treated with 100 µg/ml nitrosoguanidine (NTG) for 25 min. at room temperature without shaking. The suspensions were centrifuged, the supernatant decontaminated, and the cells were washed three times with 0.1 M potassium phosphate buffer, pH 7.0. Pellet was suspended in phosphate buffer and diluted into flasks containing 20 ml YM and shaken overnight. Cells were plated on YM and incubated at 20°C. Colonies were isolated on the basis of observed pigmentation differences, with the colonies characterized by dark reddish orange as observed under a dissecting microscope being selected, and streaked to slants. Three day old slants were used to inoculate YM flasks. One ml of YM medium was used to suspend the contents of the slant and this entire suspension was added to 250 ml flasks containing 20 ml YM and shaken for 6 days at 20°C.

Morphological Selection

Example 3: Antimycin A Treatment

Exponentially growing yeast cells of strain ATCC 24230, either NTG mutagenized as described under Step 1(a) above or non-mutagenized, were plated on YM medium containing 50 µm antimycin A (a mixture of antimycins $A_1$ and $A_3$; Sigma catalog #2006) and incubated at 20°C until a colony grew. Growth took approximately one month. After two more weeks of incubation a red center appeared in the colony presenting a fried egg appearance. This red center was isolated to YM medium.

Example 4: Tunicamycin Treatment

Tunicamycin mutants were selected by plating a log phase culture of strain ATCC 24202 (naturally occurring) on a gradient plate of GYE agar with 5 µg/ml tunicamycin. Colonies which grew on these plates were streaked to GYE agar plates containing 5 and 8 µg/ml tunicamycin. Colonies which grew on these secondary plates were assayed for pigment. Control was 270 µg/g as compared to 610 µg/g for 8 µg/ml tunicamycin resistant mutant.

Example 5: Nystatin Treatment

Nystatin mutants were isolated by inoculating log phase YM cultures of strain ATCC 24202 into YM flasks containing 3 and 5 µg/ml nystatin. After 48 hours, these selective cultures were plated to antibiotic plates containing YM agar with nystatin (3 and 5 µg/ml). Colonies obtained were assayed for pigment. Control was 270 µg/g as compared to 400 µg/g for highest nystatin mutant.

Example 6: Mevalonic Acid Treatment

Strain ATCC 24202 was mutagenized with NTG to 50% survival and plated to YM medium. Colonies were randomly plated to YM agar containing 5 mM mevalonic acid lactone. All of these colonies were subsequently transferred to medium containing first 10 and then 25 mM mevalonic acid lactone. Colonies which grew on 25 mM mevalonic acid lactone were assayed for pigment. Control was 275 µg/g as compared to 540 µg/g for 25 mM mevalonic acid lactone resistant mutant.

Carotenoid Extraction and Analysis

Colonies may be analyzed for pigment content as follows.

After harvest from 30 ml growth medium, the cells are washed in water and resuspended in 30 ml of water. The optical density is measured to determine growth. Approx. 13 ml of the suspension is broken with 0.5 mm glass beads for 3 to 4 minutes with cooling within a Bead Beater (Bio Spec Products, Bartlesville, OK). After breakage, the bead/cell mixture is poured into a beaker and extracted 5x with 10 ml aliquots of acetone. The acetone extracts are pooled and centrifuged at 15,000 rpm for 45 min. The clear acetone supernatant is poured off the cell pellet; if the pellet contains residual pigment, it is manually ground with a glass homogenizer and further extracted with acetone.

The combined acetone extracts are combined in a separatory funnel, and approximately 10 ml of petroleum ether is added as well as a few ml of a solution of saturate NaCl to help break the emulsion. The petrol extract is collected and the acetone layer is reextracted. The petrol extracts are combined and filtered through glass wool to remove lipid globules and other particulate matter. The absorbance spectrum is recorded (the absorbance maximum of transastaxanthin in petroleum ether is 474 nm). The total carotenoid composition is calculated using the 1% extinction coefficient =2100 by the formula:

$$\text{Total carotenoid } (\mu g/g \text{ yeast}) = \frac{(\text{ml petrol})\ (\text{absorbance 474 nm})\ (100)}{(21)\ (\text{dry weight yeast } [g])}$$

Individual carotenoids may be analyzed by thin layer chromatography (TLC) and electronic absorption spectra. To prepare carotenoid extracts for analysis, petroleum ether extracts are dried over anhydrous $Na_2SO_4$ and concentrated by evaporation in a stream of nitrogen. These are chromatographed by TLC on silica gel plates (Silica gel 60, 5 x 20 dm, 0.25mm thickness (E. Merck, Darmstadt, West Germany) using 20% acetone/80% petroleum ether. After development, bands are scraped and eluted in acetone through a Pasteur pipet plugged with glass wool. Absorbance maxima, $r_f$ values, and cochromatography with standards are used for identification of the pigments. Visible absorption spectra are recorded in acetone or petrol on a Gilford Response spectrophotometer, and concentrations of carotenoids are calculated using the specific absorption coefficients listed by Davies, Carotenoids, p. 38-165, in Chemistry and Biochemistry of Plant Pigments, vol. 2., T.W. Goodwin (ed.), Academic Press, London (1976).

Bench Scale Fermentation

Example 7

The production of astaxanthin in a 10 liter laboratory fermenter is illustrated by the following procedure:

20 ml. of frozen Phaffia rhodozyma strain IGI-887J1 (see Fig. 1 and accompanying text) was used to inoculate 300 ml of a medium comprising 3% glucose, 1% yeast extract and 0.1% caesin hydrolysate. The medium was sterilized at 121°C and 1 bar (15 p.s.i) for about 20 minutes before being inoculated. The inoculated medium was shaken for = 48 hours at a temperature of 20°C on a rotary shaker with a 25 mm (1 inch) stroke at a speed of 250 r.p.m. The 300 ml cell broth was used to inoculate a 5 liter seed fermenter, containing 3 liters of sterilized seed medium having the following composition per liter:

9

|  | G |
|---|---|
| Ammonium sulphate | 5.0 |
| Potassium phosphate monobasic | 1.5 |
| Magnesium sulphate heptahydrate | 1.5 |
| Calcium chloride dihydrate | 0.1 |
| Yeast extract | 6.0 |
| Vitamin mixture | 2 ml |
| Trace element mixture | 1 ml |

The vitamin mixture consists of the following ingredients:

|  | G |
|---|---|
| Biotin | 0.08 |
| Inositol | 5.00 |
| Thiamine | 5.00 |
| Calcium Pantothenate | 0.00 |
| Pyroxidine HCL | 2.25 |
| Water, q.s. to make 1L | |

The trace element mixture consists of the following ingredients:

|  |  | G |
|---|---|---|
| 1) | $FeCl_3 \cdot gH_2O$ | 3 |
| 2) | $Na_2MoO_4 \cdot 2H_2O$ | 2 |
| 3) | $ZnSO_4 \cdot 7H_2O$ | 8 |
|  | $MnSO_4 \cdot H_2O$ | 3 |
|  | $CuSO_4 \cdot 5H_2O$ | 6 |
|  | Water, q.s. to make 1L | |

After inoculation, a 70% cerelose solution was fed incrementally to the fermenter, specific growth rate of the cells was kept at about u=0.15. The fermenter was maintained at a temperature of 20°C.

Example 8

P. rhodozyma strains used were the natural isolate UCD-FST- 67-385 (Phaff et al., 1972; Miller et al., 1976), mutant Ant-1-4 used above, and strain 18-13-6, an astaxanthin enhanced mutant obtained by ethylmethane sulfonate (EMS) mutagenesis procedures (isolated on YM agar). They were grown in yeast extract/malt extract/peptone/glucose medium (YM medium, Difco Co., Detroit, MI) as previously described (An et al., 1989) in a temperature controlled incubator/ shaker (Environ-Shaker Model 3597, Lab-Line Instruments, Inc., Melrose Park, IL). Growth was determined by the optical density (660 nm) of a washed cell suspension; 1 mg dry cell weight per ml corresponds to an O.D. of 1.35.

Light was supplied by two Sylvania 20 watt Coolwhite fluorescent tubes held 20-40 cm from the flask media surfaces. Flasks were inoculated with approximately $10^2$-$10^6$ cells of actively growing yeast. For dark controls, flasks were wrapped in aluminum foil. When insoluble chemicals were included in the media, they were first dissolved in a small quantity of ethanol (0.3% final conc.), which did not affect yeast growth or pigmentation.

Carotenoid Extraction and Analysis

P. rhodozyma was grown for 5 days in flasks before extraction. Yeasts were harvested from liquid media by centrifugation. The yeast cells were suspended in distilled water, washed in water, and extracted and analyzed for carotenoids by thin-layer chromatography and absorption spectroscopy as previously described (An et al., 1989).

Secondary Respiratory Pathway Induction Increases Carotenoid Production

The influence of two 20 watt fluorescent bulbs placed 20 cm from the surface of P. rhodozyma natural isolate, UCD-FST-67-385, and its antimycin A sensitive mutants, Ant 1-4 and 18-13-6 were studied.

Separate cultures were grown under conditions of (a) total darkness for 30 hours (hereafter "dark"), (b) total darkness with 0.2 μM antimycin A (antimycin A being introduced at inoculation) (hereafter "dark/antimycin"), and under conditions (c) and (d) which were identical to (a) and (b) except for exposure of the cultures to light from approximately 30 hours into the experiment until approximately 60 hours into the experiment (hereafter 'light" and "light/ antimycin").

Extraction of the pigments and characterization indicated that the pigments were qualitatively similar in composition, except that more cis-astaxanthin and lower carotene concentrations were found to be present in light grown cells.

Analysis of yeasts showed that the carotenoid content of light/antimycin A P. rhodozyma UCD-FST-67-385 increased by two-fold over any of the dark, dark-antimycin A or light cultures of this natural isolate (Table 1). Table 2 shows that mutant 18-13-6, which is antimycin A sensitive, produced two-fold increase in carotenoid content in antimycin/light over the light culture.

Table 1

| Conditions | Growth (mg/ml) | Carotenoid (μg/g y) |
|---|---|---|
| Dark | 4.4 | 520 |
| Dark + Ant | 1.6 | 480 |
| Light | 3.3 | 440 |
| Light + Ant | 2.2 | 1000 |

Table 2

| Antimycin | Not added | Added |
|---|---|---|
| Light | Carotenoid Content (μg carotenoids/g yeast) | |
| White | 540 | 1410 |
| Blue | 1050 | 1360 |
| Red | 960 | 1210 |
| Dark | 830 | 1013 |

As can be seen from the above results, growth and carotenoid formation of natural and astaxanthin enhanced P. rhodozyma is clearly influenced by light. The natural isolate, UCD-FST-67-385, and its antimycin A-sensitive mutants ant-1-4 and 18-13-6, each grew better and had increased pigmentation in the dark, but were differently affected by light.

Phaffia As a Food and Pigment Source

The harversted cells can be disrupted or broken using mechanical or enzymatic means or a combination thereof and fed to salmonids, etc. as part of an overall nutritious diet. In order to enhance the stability of astaxanthin in the disrupted or broken yeast against heat and air, it is preferred to provide a protective coating or matrix as conventionally provided in the food art, for example, a natural gum.

Examples wherein P. rhodozyma is used as a food supplement for rainbow trout (Salmo gairdneri), American lobsters, Coturnix quail, and laying hens are discussed in a thesis of co-inventor Johnson entitled "Astaxanthin Production by the Yeast Phaffia rhodozyma and Its Use As a Pigment Source in Animal Feeding", Master's Thesis, University of California at Davis (1976), a copy submitted herewith.

As a consequence of the higher astaxanthin content per unit of cells on a dry basis in the yeast attained according to the present invention, the amount of yeast employed to induce pigmentation can be substantially reduced.

Variations on the design or operation of the above illustrative embodiments may be readily made to adapt the inventive process to various operational demands.

**Claims**

1. A process for the production of a yeast having an enhanced astaxanthin content comprising culturing, in a nutrient medium containing an antibiotic selected from antimycin A, tunicamycin and nystatin, a cytochrome B inhibitor, or a terpenoid synthetic pathway inhibitor, a microorganism of genus <u>Phaffia</u>.

2. A process as set forth in claim 1, wherein said cytochrome B inhibitor is selected from the group consisting of antimycin A and 2-n-heptyl-4-hydroxyquinoline-N-oxide.

3. A process as in claim 1, wherein the terpenoid synthetic pathway inhibitor is mevalonic acid lactone.

4. A process as set forth in claim 1, wherein the antibiotic or terpenoid synthetic pathway inhibitor concentration in the medium is between 1 and 100 μM.

5. A process as in claim 1, wherein the microorganism of genus <u>Phaffia</u> is subject to mutagenesis either before, after, or before and after morphological selection.

6. A process as in claim 1, wherein the astaxanthin in harvested yeast is 1000 ppm or more based on dry weight of yeast cells.

7. A yeast having the identifying characteristics of <u>Phaffia</u>, said yeast being obtainable by at least one step of morphological selection of naturally occurring <u>Phaffia</u>, or of a mutant thereof, cultured using a medium containing an antibiotic selection agent selected from antimycin A, tunicamycin and nystatin, a cytochrome B inhibitor or a terpenoid synthetic pathway inhibitor.

8. A process for increasing the pigmentation of the flesh of salmonids or the skin, flesh or egg yolk of fowl which comprises feeding said salmonids or fowl a yeast obtained by the process of claim 1 in disrupted form in sufficient amount to increase the pigmentation of the flesh of said salmonids or said skin, flesh or egg yolk of said fowl.

9. A process for <u>in vivo</u> production and recovery of astaxanthin comprising culturing one or more times, in a nutrient medium containing an antibiotic selected from antimycin A, tunicamycin and nystatin, a cytochrome B inhibitor, or a terpenoid synthetic pathway inhibitor, a microorganism of genus <u>Phaffia</u>, cultivating surviving microorganisms exhibiting enhanced pigmentation, harvesting the cultivated yeast, and extracting the astaxanthin.

10. A process as in claim 9 further comprising subjecting said microorganism of genus <u>Phaffia</u> to at least one mutation either before or after one of said culturings in said nutrient medium containing an antibiotics, a cytochrome B inhibitor, or a terpenoid synthetic pathway inhibitor.

11. A process for the production of a yeast having an enhanced astaxanthin content comprising subjecting yeast obtained according to claim 1 to growth in the presence of a main respiratory pathway inhibitor and in the presence of an agent or under the influence of an environmental condition which triggers a secondary respiratory oxidative pathway.

**Patentansprüche**

1. Verfahren zum Herstellen einer Hefe, die einen erhöhten Astaxanthin-Gehalt hat, wobei das Verfahren aufweist: Züchten eines Mikroorganismus der Gattung <u>Phaffia</u> in einem Nährmedium, das folgendes enthält: ein Antibiotikum, das aus Antimycin A, Tunicamycin und Nystatin ausgewählt ist, einen Cytochrom-B-Hemmstoff oder einen Terpen-Syntheseweg-Hemmstoff.

2. Verfahren nach Anspruch 1, wobei der Cytochrom-B-Hemmstoff aus der Gruppe ausgewählt wird, die aus Antimycin A und 2-n-Heptyl-4-hydroxychinolin-N-oxid besteht.

3. Verfahren nach Anspruch 1, wobei der Terpen-Syntheseweg-Hemmstoff Mevalolacton ist.

4. Verfahren nach Anspruch 1, wobei die Konzentration des Antibiotikums oder des Terpen-Syntheseweg-Hemmstoffs in dem Medium zwischen 1 und 100 μM liegt.

5. Verfahren nach Anspruch 1, wobei der Mikroorganismus der Gattung <u>Phaffia</u> entweder vor oder nach oder vor und nach der morphologischen Selektion der Mutagenese ausgesetzt wird.

**6.** Verfahren nach Anspruch 1, wobei das Astaxanthin in geernteter Hefe 1000 ppm oder mehr, bezogen auf das Trockengewicht von Hefezellen, ist.

**7.** Eine Hefe, die die kennzeichnenden Charakteristiken von Phaffia hat, wobei die Hefe erhältlich ist durch wenigstens einen Schritt der morphologischen Selektion von natürlich vorkommender Phaffia oder einer Mutante davon, die gezüchtet ist unter Einsatz eines Mediums, das ein antibiotisches Selektionsmittel, das aus Antimycin A, Tunicamycin und Nystatin ausgewählt ist, oder einen Cytochrom-B-Hemmstoff oder einen Terpen-Syntheseweg-Hemmstoff enthält.

**8.** Verfahren zur Steigerung der Pigmentierung des Fleisches von Salmoniden oder der Haut, des Fleisches oder der Eidotter von Geflügel, wobei das Verfahren folgendes aufweist: Füttern der Salmoniden oder des Geflügels mit einer Hefe, die nach dem Verfahren von Anspruch 1 erhalten ist, in gebrochener Form in ausreichender Menge, um die Pigmentierung des Fleisches der Salmoniden oder der Haut, des Fleisches oder der Eidotter des Geflügels zu steigern.

**9.** Verfahren zur In-vivo-Herstellung und Gewinnung von Astaxanthin, wobei das Verfahren folgendes aufweist: einmaliges oder mehrmaliges Züchten eines Mikroorganismus der Gattung Phaffia in einem Nährmedium, das ein aus Antimycin A, Tunicamycin und Nystatin ausgewähltes Antibiotikum, einen Cytochrom-B-Hemmstoff oder einen Terpen-Syntheseweg-Hemmstoff enthält, Kultivieren von überlebenden Mikroorganismen, die verstärkte Pigmentierung zeigen, Ernten der kultivierten Hefe und Extrahieren des Astaxanthins.

**10.** Verfahren nach Anspruch 9, das ferner folgendes aufweist: Unterwerfen des Mikroorganismus der Gattung Phaffia wenigstens einer Mutation entweder vor oder nach einem der Züchtungsvorgänge in dem Nährmedium, das ein Antibiotikum, einen Cytochrom-B-Hemmstoff oder einen Terpen-Syntheseweg-Hemmstoff enthält.

**11.** Verfahren zur Herstellung einer Hefe, die einen erhöhten Astaxanthin-Gehalt hat, wobei das Verfahren folgendes aufweist: Unterziehen einer nach Anspruch 1 erhaltenen Hefe dem Wachstum in Gegenwart eines Hauptatmungsweg-Hemmstoffs und in Gegenwart eines Agens oder unter dem Einfluß einer Umgebungsbedingung, das/die einen Sekundäratmungsweg triggert.

## Revendications

**1.** Procédé pour la production d'une levure ayant une teneur en astaxanthine augmentée, comportant la culture, dans un milieu nutritif contenant un antibiotique sélectionné parmi antimycine A, tunicamycine et nystatine, un inhibiteur cytochrome B ou un inhibiteur terpenoïde par voie synthétique, un microorganisme du gène Phaffia.

**2.** Procédé selon la revendication 1, dans lequel ledit inhibiteur cytochrome B est sélectionné dans le groupe constitué par antimycine A et 2-n-heptyl-4-hydroxyquinoline-N-oxyde.

**3.** Procédé selon la revendication 1, dans lequel l'inhibiteur terpénoïde par voie synthétique est lactone acide mévalonique.

**4.** Procédé selon la revendication 1, dans lequel la concentration de l'antibiotique ou l'inhibiteur terpenoïde par voie synthétique dans le milieu est comprise entre 1 et 100 μm.

**5.** Procédé selon la revendication 1, dans lequel le microorganisme du gène Phaffia est sujet à mutagénèse soit avant, après ou avant et après sélection morphologique.

**6.** Procédé selon la revendication 1, dans lequel l'astaxanthine dans la levure collectée est 1 000 ppm ou plus, basé sur le poids sec de cellules de levure.

**7.** Levure ayant les caractéristiques d'identification de Phaffia, ladite levure pouvant être obtenue par au moins une étape de sélection morphologique de Phaffia se produisant naturellement ou d'un de ses mutants, cultivée utilisant un milieu contenant un agent de sélection antibiotique sélectionné parmi antimycine A, tunicamycine et nystatine, un inhibiteur cytochrome B ou un inhibiteur terpenoïde par voie synthétique.

**8.** Procédé pour accroître la pigmentation de la chair des salmonidés, ou la peau, la chair ou le jaune d'oeuf de la volaille, qui comporte de faire absorber par lesdits salmonidés ou la volaille une levure obtenue par le procédé de la revendication 1, sous forme brisée en quantité suffisante pour accroître la pigmentation de la chair desdits salmonidés ou la peau, la chair ou le jaune d'oeuf de ladite volaille.

9. Procédé pour la production in vivo et la récupération d'astaxanthine comportant l'étape de cultiver une ou plusieurs fois, dans un milieu nutritif contenant un antibiotique sélectionné parmi antimycine A, tunicamycine et nystatine, un inhibiteur cytochrome B ou un inhibiteur terpenoïde par voie synthétique, un microorganisme du gène Phaffia, l'étape de cultiver les microorganismes survivants, présentant une pigmentation augmentée, l'étape de récolter la levure cultivée, et l'étape d'extraire l'astaxanthine.

10. Procédé selon la revendication 9, comportant en outre l'étape de soumettre ledit microorganisme du gène Phaffia à au moins une mutation soit avant ou après une desdites cultures dans ledit milieu nutritif contenant un antibiotique, un inhibiteur cytochrome B ou un inhibiteur terpenoïde par voie synthétique.

11. Procédé pour la production d'une levure ayant une teneur augmentée en astaxanthine, comportant l'étape de soumettre la levure obtenue conformément à la revendication 1, à une croissance en présence d'un inhibiteur principal par voie respiratoire et en présence d'un agent ou sous l'influence d'une condition expérimentale, qui déclenche une voie oxydante respiratoire secondaire.

ECHINENONE (A),

3-HYDROXYECHINENONE (B), PHEONICOXANTHIN (C) AND ASTAXANTHIN (D)

IN PHAFFIA RHODOZYMA

# FIG.1

67 - 385 ( ATCC Nos. 24230 )

ANTIMYCIN SELECTION

IGI - 887J0

NITROSOGUANIDINE (NTG) MUTATION

IGI - 887J2

SELECTED WITH ANTIMYCIN

IGI - 887J1

SECOND NTG MUTAGENESIS

IGI - 887 J3


IGI - 887J3

PURIFIED INTO WHITE AND PIGMENTED COLONIES.

IGI - 887J4    ( WHITE REVERTANT )

NITROSOGUANIDINE MUTATION

IGI - 1287 J1


IGI - 887J2

NITROSOGUANIDINE MUTATION

IGI - 2880B60


FIG.2

FIG.3a

FIG.3b

FIG.3c